# EUROPÄISCHE PATENTSCHRIFT

(11) **EP 2 362 153 B1**
(45) Veröffentlichungstag und Bekanntmachung des Hinweises auf die Patenterteilung: **14.06.2017**
(21) Anmeldenummer: 10185524.5
(22) Anmeldetag: 21.03.2003
(51) Int. Cl.: F24F 3/16, A61L 9/20, F24F 12/00, F24F 13/14

(54) **Luftaustauschvorrichtung**
Air exchanging device
Dispositif de renouvellement d'air

(30) Priorität: 25.03.2002 DE 20204755 U
(43) Veröffentlichungstag der Anmeldung: 31.08.2011
(62) Teilanmeldung aus: 03720228.0
(73) Patentinhaber: Meltem Wärmerückgewinnung GmbH & Co. KG, 82239 Alling (DE)
(72) Erfinder: Reynartz, Armin, 82239, Alling (DE)
(74) Vertreter: Schweiger, Martin

(56) Entgegenhaltungen:
- EP-A- 0 344 492
- EP-A2- 0 029 573
- WO-A1-93/18360
- WO-A1-97/01736
- DE-A1- 3 230 279
- DE-A1- 3 640 347
- DE-A1- 19 644 849
- US-A- 5 000 253
- US-B1- 6 345 510

## Beschreibung

Die Erfindung betrifft eine Luftaustauschvorrichtung, insbesondere für einen Innenraum eines Gebäudes. Solche Luftaustauschvorrichtungen befördern verbrauchte, warme Abluft von einer Gebäudeinnenseite auf eine Gebäudeaußenseite und gleichzeitig frische Außenluft von der Gebäudeaußenseite in das Gebäudeinnere. Diese beiden Luftströme werden oft in einem innerhalb der Luftaustauschvorrichtung vorsehbaren Wärmetauscher aneinander vorbeigeführt, um die kalte Außenluft zu erwärmen.

Bei üblichen Luftaustauschvorrichtungen kommen oft kastenartige Filter zur Reinigung der von außerhalb eines Gebäudes in das Gebäudeinnere strömenden Außenluft sowie der in gegengesetzter Richtung strömenden, verbrauchten Abluft zum Einsatz. Solche kastenartigen Filter weisen oft eine Vielzahl von nebeneinander angeordneten Rippen auf, durch welche die Außenluft und die Abluft strömen. Dabei ist von Nachteil, daß durch solche kastenartigen Filter durchströmende Luft einen großen Reibwiderstand erfährt. Dementsprechend ist der Einsatz von solchen kastenartigen Filtern mit großen Gesamtdurchströmungsverlusten verbunden, was den Wirkungsgrad und auch den Energieverbrauch von mit solchen kastenartigen Filtern versehenen Luftaustauschvorrichtungen beeinträchtigt. Weiterhin weisen solche kastenartige Filter eine große Baugröße auf und sind demzufolge unhandlich zu warten und auszuwechseln.

Gängige Luftaustauschvorrichtungen umfassen meist Lüfter für die Förderung von Zuluft in einen Innenraum eines Gebäudes. Die Zuluft wird durch diese häufig als Radial- oder Axiallüfter aufgebauten Lüfter von außerhalb des Gebäudes angesaugt. Beim Betrieb solcher Lüfter entsteht ein konstanter Geräuschpegel von durch die Bewegung des Lüfters verursachten Geräuschen. Zumeist sind diese Lüfter unmittelbar an einer Öffnung angebracht, durch welche die Zuluft in das Gebäudeinnere strömt. Dadurch ergibt sich der Nachteil, daß der Geräuschpegel des Lüfters im Innenraum des Gebäudes deutlich wahrnehmbar ist und von sich dort aufhaltenden Personen als störend empfunden wird.

Es ist daher Aufgabe der Erfindung, eine geräuscharme Luftaustauschvorrichtung anzugeben, mit der durchströmende Luft effektiv gereinigt werden kann.

Diese Aufgabe wird durch den Gegenstand der unabhängigen Ansprüche gelöst. Vorteilhafte Ausgestaltungen ergeben sich aus den jeweiligen Unteransprüchen.

Gemäß der Erfindung ist eine Luftaustauschvorrichtung, insbesondere für einen Innenraum eines Gebäudes, vorgesehen, die einen Ablufteinlassbereich aufweist. In diesen Ablufteinlassbereich strömt verbrauchte, warme Luft aus den Innenraum.

Eine besonders vorteilhafte Strömung der Abluft ist dann gegeben, wenn bei der erfindungsgemäßen Abluftfiltervorrichtung eine Abluftzuführungswand bzw. eine Leitwand angeordnet ist, die eine der Strömungsrichtung der Abluft entgegengesetzte Leitrippe aufweist. Dabei ist die Anströmungsrichtung der Abluft senkrecht zu der länglichen Symmetrieachse der Abluftfiltervorrichtung gerichtet.

Die Erfindung betrifft ferner eine Luftaustauschvorrichtung mit einem Gehäuse und mit einem elektrischen Lüfter, der in diesem Gehäuse angeordnet ist und der Zuluft in den Innenraum befördert. An der Zuluftauslassöffnung dieses Gehäuses setzt ein Zuluftführungskanal an und verläuft entlang des Bereichs wenigstens einer Gehäuseseite parallel zu der Gehäusevorderseite, bevor die Zuluft aus einem Zuluftauslassbereich des Zuluftführungskanals in das Gebäudeinnere austritt. Dadurch wird eine Schalldämpfung der durch den Lüfter erzeugten Drehgeräusche bewirkt. Die auf die Innenseite des Gebäudes gelangenden Geräusche werden durch das Vorsehen solch eines Zuluftführungskanals in der Lautstärke deutlich vermindert. Die Schalldämpfungswirkung des Zuluftführungskanals entspricht prinzipiell derjenigen eines Motorauspuffs.

Dabei ist ein Zuluftführungskanal vorsehbar, der ein Duftstoffbehältnis in einem Bereich aufweist. An diesem Duftstoffbehältnis strömt die Zuluft vorbei und nimmt Teile des Duftstoffs auf. Der Duftstoff kann dabei als Flüssigkeit, insbesondere als Duftöl oder in Pulverform, vorliegen. Durch das Vorsehen eines solchen Duftstoffbehältnisses an einem Zuluftführungskanal kann auf einfache Weise eine Verbesserung des Geruchs und der wahrgenommenen Qualität der Zuluft erreicht werden.

Anstelle des Duftstoffbehältnisses oder zusätzlich dazu kann auch ein Zerstäuber, insbesondere ein Ultraschallzerstäuber, zum Zerstäuben von separat zugeleitetem Wasser und/oder von Kondenswasser vom Wärmetauscher und/oder ein Kapillaren aufweisender Keramikzylinder zum Verdampfen von Duftstoffen, insbesondere von Duftöl, in einem Bereich des Zuluftführungskanals vorgesehen sein. Zur Luftbefeuchtung werden definierte Mengen von Wasser durch den Ultraschallzerstäuber in Richtung des Zuluftführungskanals zerstäubt. Dadurch wird eine optimale Luftfeuchtigkeit der Zuluft gewährleistet, die über die Menge des durch den Zerstäuber an die Zuluft abgebbaren Wassers genau einstellbar ist. Durch den Keramikzylinder werden Duftstoffe infolge seiner Kapillarwirkung kontinuierlich verdampft und an die im Zuluftführungskanal vorbeiströmende Luft abgegeben. Durch den Einsatz eines solchen Keramikzylinders zum Verdampfen von Duftstöffen kann die Konzentration der Duftstoffe und somit der Geruch der Zuluft besonders zuverlässig und präzise eingestellt werden.

Gemäß einer weiteren vorteilhaften Weiterbildung der Erfindung weist der Zuluftführungskanal eine im wesentlichen parallel zur Luftströmungsrichtung verlaufende Zwischenwandung auf. Diese ist insbesondere mittig in dem Zuluftführungskanal angeordnet. Durch das Vorsehen solch einer Zwischenwandung werden die Strömungsverluste in dem Zuluftführungskanal reduziert. Durch die derartige Verbesserung der Luftströmung in dem Zuluftführungskanal wird ein erhöhter Wirkungsgrad der Luftaustauschvorrichtung erreicht. So wird ein energiesparender Betrieb der Luftaustauschvorrichtung, insbesondere mit einer reduzierten Umdrehungszahl des Lüfterrads bzw. der Lüfterräder, ermöglicht. Die eingesetzten Lüfter können so mit einer sehr niedrigen geräusch- und energieverbrauchsoptimierten Drehzahl betrieben werden. Durch die verbesserte Luftströmung kann auch ein schlechterer Wirkungsgrad des Wärmetauschers ausgeglichen werden.

Noch vorteilhafter ist die Schalldämpfung, wenn der Zuluftführungskanal bogenförmig ausgebildet ist und sich entlang der Bereiche von zwei aneinandergrenzenden Gehäuseseiten erstreckt, wie es gemäß einer vorteilhaften Weiterbildung vorgesehen ist.

Die US 5,000,253 offenbart ein zentrales Wärmerückgewinnungs- und Ventilationssystem mit einem Wärmetauscher in dem gerade Luftkanäle durch bogenförmige Kanalabschnitte miteinander verbunden sind, wobei die bogenförmigen Kanalabschnitte einen Stromteiler aufweisen, der gemäß der US 5,000,253 eine zu starke Konzentration der Luftmassen an der Außenseite des Bogens verhindert.

Mittels einer innerhalb des Zuluftführungskanals oder in einem Bereich des Zuluftführungskanals angeordneten UV-Lampe können in der Zuluft vorhandene Keime abgetötet werden, wodurch eine verbesserte Qualität der Zuluft sichergestellt wird.

Die Erfindung betrifft ferner eine Luftaustauschvorrichtung mit einer Abluftklappe zum selektiven Verschließen eines Ablufteinlasses sowie mit einer Zuluftklappe zum selektiven Verschließen eines Zuluftauslasses. Dabei ist erfindungsgemäß genau ein Elektromotor vorgesehen, der mittels eines Antriebsritzels eine Betätigungsmechanik sowohl für die Abluftklappe als auch für die Zuluftklappe betreibt. Die Zuluftklappe ist dabei derart mit einer Zuluftklappen-Drehachse verbunden, daß sie drehbar ist und zwar jeweils begrenzt durch Anschlagsbereiche zwischen einer Zuluftklappen-Schließstellung und einer Zuluftklappen-Öffnungsstellung. Die Abluftklappe ist derart mit einer Abluftklappen-Drehachse verbunden, daß drehbar ist und zwar jeweils begrenzt durch Anschlagsbereiche zwischen einer Abluftklappen-Schließstellung und einer Abluftklappen-Öffnungsstellung.

Der Drehwinkel der Zuluftklappe zwischen ihrer Zuluftklappen-Schließstellung und ihrer Zuluftklappen-Öffnungsstellung ist verschieden vom Drehwinkel der Abluftklappe zwischen ihrer Abluftklappen-Schließstellung und ihrer Abluftklappen-Öffnungsstellung. Zwischen der Zuluftklappen-Drehachse und der Abluftklappen-Drehachse, die miteinander lösbar verbunden sind, ist ein Spiel vorgesehen, das eine Verdrehbarkeit der Abluftklappen-Drehachse bezüglich der Zuluftklappen-Drehachse gewähr-leistet. Die Verbindung zwischen der Zuluftklappen-Drehachse und der Abluftklappen-Drehachse kann als bajonettförmige Kopplung vorgesehen sein.

In dem Fall, daß der Abluftklappenwinkel größer ist als der Zuluftklappenwinkel, greift das Antriebsritzel des Elektromotors an einem Ritzelbereich eines Ritzels der Abluftklappen-Drehachse an. Falls der Abluftklappenwinkel kleiner ist als der Zuluftklappenwinkel, greift das Antriebsritzel des Elektromotors an einem Ritzelbereich eines Ritzels der Zuluftklappen-Drehachse an. Der Zuluftklappenwinkel ergibt sich dabei aus dem Winkel zwischen der Zuluftklappen-Schließstellung und der Zuluftklappen-Öffnungsstellung. Der Abluftklappenwinkel berechnet sich aus der Differenz zwischen der Abluftklappen-Schließstellung und der Abluftklappen-Öffnungsstellung.

Ein erfindungsgemäßer Grundgedanke besteht dabei darin, daß das Spiel in der Verbindung der beiden Drehachsen genauso groß ist wie die Differenz zwischen dem Zuluftklappenwinkel und dem Abluftklappenwinkel. Dadurch wird gewährleistet, daß der Elektromotor beim Erreichen des Anschlags durch die erste Klappe die zweite Klappe noch weiter drehen kann.

Durch diese Ausführungsform der Erfindung wird eine besonders vorteilhafte und energiesparende Steuerung von zwei Klappen mit einem einzigen Motor gewährleistet. Für die Zuluftklappe ist nämlich oft eine in Bezug auf die Zuluftströmungsrichtung waagrechte Position optimal, wobei für die Abluftklappe eine in Bezug auf die Strömungsrichtung der Abluft leicht geneigte Stellung vorteilhaft ist. Die Schließstellung ist bei der Abluftklappe und bei der Zuluftklappe jedoch identisch. Dementsprechend ergibt sich beim erfindungsgemäßen Luftaustauschvorrichtung für die Abluftklappe und für die Zuluftklappe jeweils ein unterschiedlicher Winkel zwischen ihrer Öffnungs- und Schließstellung.

Durch die Erfindung ist auch eine Luftaustauschvorrichtung mit einer Abluftklappe und mit einer Zuluftklappe umfaßt, wobei je eine durch das Antriebsritzel eines Elektromotors angetriebene Betätigungsmechanik für die Abluftklappe und für die Zuluftklappe, ein Energiespeicher sowie eine Ansteuerschaltung für den Elektromotor bzw. für die Elektromotoren vorgesehen ist. Der Energiespeicher kann dabei in elektrischer Form als Batterie, als Kondensator oder als Spule oder in mechanischer Form, wie beispielsweise als Feder, vorgesehen sein. Die Ansteuerungsschaltung ist dabei so ausgebildet, daß bei einer Unterbrechung der Energiezufuhr zu der Luftaustauschvorrichtung, insbesondere bei einem Stromausfall, der Elektromotor bzw. die Elektromotoren so ansteuerbar sind, daß die im Energiespeicher vorhandene Energie genutzt wird, indem die Zuluftklappe und die Abluftklappe jeweils in eine Abluftklappen-schließstellung gedreht werden können.

Dadurch ist auf einfache und effiziente Weise gewährleistet, daß die Luftaustauschvorrichtung bei einer Unterbrechung der Energiezufuhr, insbesondere bei einem Stromausfall, in einen geschützten Zustand mit jeweils geschlossenen Klappen gebracht wird. Damit wird einem Eindringen von Zugluft in das Gebäudeinnere entgegengewirkt, wenn der Strom ausfällt.

Die Erfindung ist in den Zeichnungen anhand eines Ausführungsbeispiels näher veranschaulicht.
- Figur 1: zeigt eine schematische Darstellung einer Draufsicht auf eine Luftaustauschvorrichtung gemäß einem Ausführungsbeispiel,
- Figur 2: zeigt eine schematische Darstellung eines Querschnitts einer an einer Außenwand eines Gebäudes befestigten Luftaustauschvorrichtung gemäß Figur 1,
- Figur 3: zeigt eine schematische Darstellung eines weiteren Querschnitts der Luftaustauschvorrichtung gemäß Figur 1,
- Figur 4: zeigt eine schematische, perspektivische Darstellung einer auf die Vorderseite eines Gehäuses der Luftaustauschvorrichtung gemäß Figur 1 aufbringbaren Abdeckplatte,
- Figur 5: zeigt eine Prinzipskizze einer Klappenbetätigungsmechanik einer Abluftklappe und einer Zuluftklappe der Luftaustauschvorrichtung gemäß Figur 1,
- Figur 6: zeigt eine schematische Darstellung eines Schnitts der Abdeckplatte aus Figur 4,
- Figur 7: zeigt eine schematische Darstellung eines weiteren Schnitts der Abdeckplatte aus Figur 4 und
- Figur 8: zeigt eine schematische Darstellung der Luftströmungen in einem Ablufteinlassbereich der Luftaustauschvorrichtung gemäß Figur 1.

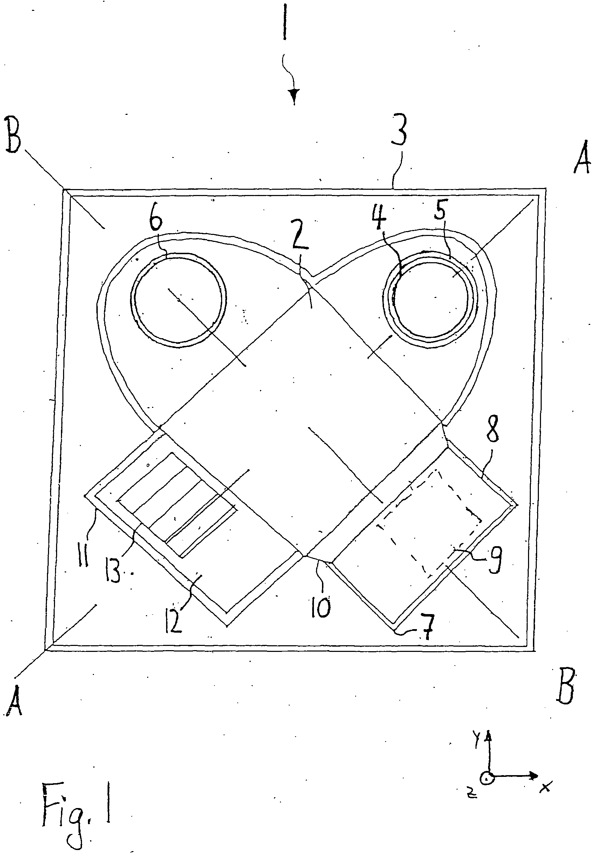

Figur 1 zeigt eine schematische Darstellung einer Draufsicht auf eine Luftaustauschvorrichtung 1 gemäß einem Ausführungsbeispiel.

Die Begriffe "unten" und "oben" werden hier so zur Beschreibung verwendet, wie es in Figur 1 mittels der y-Achse eines Koordinatensystems veranschaulicht ist. Die Begriffe "vorne" und "hinten" werden hier in Richtung der z-Achse gesehen verwendet. Die Begriffe "rechts" und "links" werden in Richtung der x-Achse gesehen verwendet.

Die Luftaustauschvorrichtung 1 ist von einem quadratischen Gehäuse 3 umschlossen. Bei der in Figur 1 gezeigten Draufsicht ist die Abdeckplatte des Gehäuses 3 abgenommen. Das Gehäuse 3 weist im Ausführungsbeispiel Kunststoff auf, ist aber auch aus anderen Materialien fertigbar.

Mittig in dem Gehäuse 3 ist ein quadratischer Plattenwärmetauscher 2 angeordnet, insbesondere aus gut wärmeleitendem Material, wobei in der Draufsicht gemäß Figur 1 nur die oberste Platte des Plattenwärmetauschers 2 sichtbar ist. Die Seitenlänge des Plattenwärmetauschers 2 entspricht knapp der Hälfte der Seitenlänge des Gehäuses 3. Der Plattenwärmetauscher 2 ist in einem Winkel von 45° bezogen auf das Gehäuse 3 gedreht angeordnet.

Oberhalb der rechten oberen Seite des Plattenwärmetauschers 2 ist eine zylinderförmige Außenluft-Rundfilterpatrone 5 im wesentlichen zentriert zum Plattenwärmetauscher 2 angeordnet. Die Außenluft-Rundfilterpatrone 5 liegt mit ihrem unteren Rand auf der Unterseite des Gehäuses 3 auf. In der in Figur 1 gezeigten Draufsicht auf die Außenluft-Rundfilterpatrone 5 ist nur deren kreisringförmiger, dünnwandiger Mantelbereich erkennbar. Beim Aufbringen einer hier nicht gezeigten Abdeckplatte auf das Gehäuse 3 liegt die Unterseite der Abdeckplatte auf der kreisringförmigen Oberseite der Außenluft-Rundfilterpatrone 5 auf.

Im Inneren der Außenluft-Rundfilterpatrone 5 ist eine kreisrunde Außenlufteinlassöffnung 4 sichtbar, die sich auf der Unterseite des Gehäuses 3 befindet. Der Durchmesser der Außenlufteinlassöffnung 4 ist geringfügig kleiner ausgebildet als der Innendurchmesser der Außenluft-Rundfilterpatrone 5. Der kleinere Durchmesser der Außenlufteinlassöffnung 4 ist zur besseren Sichtbarmachung der Außenlufteinlassöffnung 4 gewählt. Vorzugsweise ist der Durchmesser der Außenlufteinlassöffnung 4 gleich dem Innendurchmesser der Außenluft-Rundfilterpatrone 5.

Um die Außenluft-Rundfilterpatrone 5 ist eine auf der Unterseite des Gehäuses 3 aufsetzende, gekrümmte Wand vorgesehen, die am oberen sowie am unteren Ende der rechten oberen Seite des Plattenwärmetauschers 2 abschließt.

Auf der linken oberen Seite des Plattenwärmetauschers 2 ist eine ähnlich geformte Wand vorgesehen, die am oberen sowie am unteren Ende der linken oberen Seite des Plattenwärmetauschers 2 abschließt.

Innerhalb dieser Wand ist eine Abluft-Rundfilterpatrone 6 in etwa zentriert angeordnet. Die Abluft-Rundfilterpatrone 6 entspricht hinsichtlich der Form und hinsichtlich der Größe der Außenluft-Rundfilterpatrone 5. In der in Figur 1 gezeigten Draufsicht auf die Abluft-Rundfilterpatrone 6 ist nur deren kreisringförmiger, dünnwandiger Mantelbereich erkennbar. In der hier nicht gezeigten Abdeckplatte des Gehäuses 3 ist eine Öffnung vorgesehen, die beim Aufsetzen der Abdeckplatte auf das Gehäuse 3 innerhalb der Abluft-Rundfilterpatrone 6 angeordnet ist, wobei die Unterseite der Abdeckplatte auf der kreisringförmigen Oberseite der Abluft-Rundfilterpatrone 6 aufliegt.

Die Außenluft-Rundfilterpatrone 5 sowie die Abluft-Rundfilterpatrone 6 weisen in ihrem Mantelbereich filterndes Material auf, insbesondere Filterpapier oder Aktivkohle.

An der rechten unteren Seite des Plattenwärmetauschers 2 ist ein Fortluft-Lüftergehäuse 7 mit einem Fortluftradiallüfter 8 sowie ein zwischen dem Fortluftradiallüfter 8 und dem Plattenwärmetauscher 2 angeordneter Trichter 10 vorgesehen. Der Trichter 10 setzt auf der gesamten Breite der rechten unteren Seite des Plattenwärmetauschers 2 an und verjüngt sich auf einer Strecke etwa einem Achtel der Seitenlänge des Plattenwärmetauschers 2 um einen Winkel von 30°. Auf der gesamten Breite des Auslassseite des Trichters 10 setzt der Fortluftradiallüfter 8 an, von dem in der Draufsicht in Figur 1 lediglich die Verkleidung erkennbar ist. Die Drehachse des Fortluftradiallüfters 8 ist dabei senkrecht auf der Höhe der Mitte der rechten unteren Seite des Plattenwärmetauschers 2 ausgerichtet. Der Durchmesser bzw. die Breite des Fortluftradiallüfters 8 entspricht dabei in etwa der Auslassseite des Trichters 10. Die Tiefe des Fortluftradiallüfters 8 entspricht ungefähr der Hälfte seiner Breite. Der Fortluftradiallüfter 8 ist von einem Fortluft-Lüftergehäuse 7 umschlossen, das auf seiner Unterseite eine in Figur 1 gestrichelt dargestellte, rechteckige Fortluftauslassöffnung 9 aufweist. Diese Fortluftauslassöffnung 9 ist dabei von der Mitte des Fortluftradiallüfters 8 ausgehend etwas nach rechts oben versetzt angeordnet.

An der linken unteren Seite des Plattenwärmetauschers 2 setzt ein von einem dünnwandigen Zuluft-Lüftergehäuse 11 umschlossener Zuluftansaugradiallüfter 12 an. Dabei ist die Drehachse des Zuluftansaugradiallüfters 12 orthogonal zu der linken unteren Seite des Plattenwärmetauschers 2 auf der Höhe deren Mitte angeordnet. Der Durchmesser des Zulaufansaugradiallüfters 12 bzw. seine Breite entspricht in etwa der Seitenlänge des Plattenwärmetauschers 2, seine Tiefe entspricht ungefähr der Hälfte seiner Breite. In dem linken oberen Bereich des Zuluftansaugradiallüfters 12 ist eine rechteckige Zulufteinlassöffnung 13 angeordnet, durch die mehrere Lüfterschaufeln des Zuluftansaugradiallüfters 12 erkennbar sind. Diese sind im Ausführungsbeispiel parallel zur Drehachse des Zuluftansaugradiallüfters 12 angeordnet, können jedoch auch schräg zu dieser oder gekrümmt verlaufen.

Ein erste Schnittlinie A-A verläuft durch die linke untere sowie durch die rechte obere Ecke des Gehäuses 3. Eine weitere Schnittlinie B-B verläuft durch die linke obere sowie durch die rechte untere Ecke des Gehäuses 3.

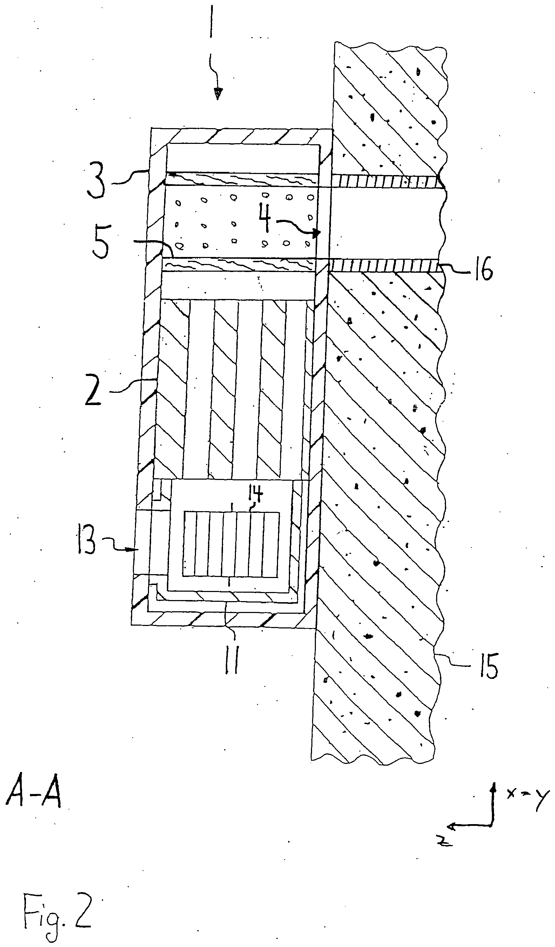

Figur 2 zeigt eine schematische Darstellung eines Querschnitts entlang der Schnittlinie A-A einer an einer Außenwand 15 befestigten Luftaustauschvorrichtung 1 gemäß dem Ausführungsbeispiel. In Figur 2 ist die Erwärmseite des Plattenwärmetauschers 2 betrachtet.

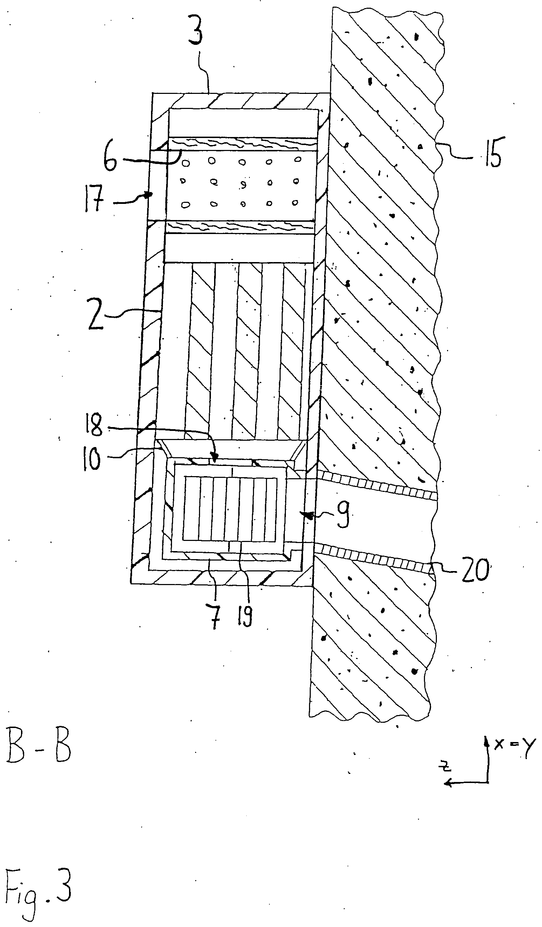

Die Luftaustauschvorrichtung 1 liegt mit der Rückseite seines Gehäuses 3 an der Vorderseite der Außenwand 15 an und befindet sich dementsprechend im Gebäudeinneren. Aus der Querschnittsdarstellung in Figur 3 ist ersichtlich, daß die diagonale Höhe der Luftaustauschvorrichtung 1 in etwa dem Dreifachen seiner Tiefe entspricht. Dabei gliedert sich das Innere des Gehäuses 3 in einen zuoberst angeordneten Außenlufteinlassbereich, in einen mittig angeordneten Plattenwärmetauscherbereich und in einen zuunterst angeordneten Zuluftauslassbereich.

In dem Außenlufteinlassbereich ist an der Rückseite des Gehäuses 3 die Außenlufteinlassöffnung 4 ausgebildet, an die sich außenwandseitig ein waagerechtes Außenluftzufuhrrohr 16 anschließt. Der Außenlufteinlassbereich umfaßt auch die Außenluft-Rundfilterpatrone 5, deren Mantelbereich sich waagerecht zwischen der Vorderseite und der Rückseite des Gehäuses 3 erstreckt und jeweils mit der Vorderseite und mit der Rückseite des Gehäuses 3 abschließt. Der mit der Rückseite des Gehäuses 3 abschließende Bereich der Außenluft-Rundfilterpatrone 5 umgrenzt dabei die Außenlufteinlassöffnung 4. Der Fasermaterial, insbesondere Aktivkohle aufweisende Mantelbereich der Au-ßenluft-Rundfilterpatrone 5 ist in Figur 2 besonders gut erkennbar.

In dem Plattenwärmetauscherbereich ist der Plattenwärmetauscher 2 angeordnet, der drei parallele, in der Darstellung gemäß Figur 3 von oben nach unten verlaufende Luftführungen aufweist. Der hier gezeigte Plattenwärmetauscher 2 entspricht prinzipiell dem Plattenwärmetauscher 2 aus Figur 2, weist jedoch acht übereinander angeordnete Platten auf. In dem Zuluftauslassbereich ist ein Zuluft-Lüfterrad 14 innerhalb des Zuluft-Lüftergehäuses 11 angeordnet. Das Zuluft-Lüfterrad 14 ist dabei horizontal mittig angeordnet. Auf gleicher Höhe mit den Lüfterschaufeln des Zuluft-Lüfterrads 14 ist in der Vorderseite des Gehäuses 3 sowie in der linken Seite des Zuluft-Lüftergehäuses 11 eine Zuluftauslassöffnung 13 vorgesehen, deren Höhe der Breite der Lüfterschaufeln entspricht.

Das Zuluft-Lüftergehäuse 11 setzt auf dem linken sowie auf dem rechten Ende der Unterseite des Plattenwärmetauschers 2 an, verläuft beidseitig vertikal nach unten und weist unmittelbar vor der Unterseite des Gehäuses 3 eine waagerechte Unterseite auf.

Figur 3 zeigt eine schematische Darstellung eines Querschnitts entlang der Schnittlinie B-B der an der Außenwand 15 befestigten Luftaustauschvorrichtung 1 gemäß dem Ausführungsbeispiel. In Figur 3 ist die Abkühlseite des Plattenwärmetauschers 2 betrachtet.

Das Innere des Gehäuses 3 gliedert sich dabei in einen oberen Ablufteinlassbereich (22), in einen mittleren Plattenwärmetauscherbereich und in einen unteren Fortluftauslassbereich. Der Ablufteinlassbereich (22) beinhaltet die Abluft-Rundfilterpatrone 6, deren Mantelbereich sich waagerecht zwischen der Vorderseite und der Rückseite des Gehäuses 3 erstreckt und jeweils mit der Vorderseite und mit der Rückseite des Gehäuses 3 abschließt. Im Ablufteinlassbereich (22) ist auf der Vorderseite des Gehäuses 3 eine Ablufteinlassöffnung 17 vorgesehen, die von der Abluft-Rundfilterpatrone 6 umgrenzt wird. Der Fasermaterial, insbesondere Aktivkohle aufweisende Mantelbereich der Abluft-Rundfilterpatrone 6 ist in Figur 3 besonders gut zu sehen.

Im Plattenwärmetauscherbereich ist der Plattenwärmetauscher 2 angeordnet, der vier parallele, vertikal verlaufende Luftführungen aufweist. Der hier gezeigte Plattenwärmetauscher 2 entspricht prinzipiell dem Plattenwärmetauscher 2 aus Figur 2, weist jedoch acht übereinander angeordnete Platten auf. Der Fortluftauslassbereich umfaßt einen hinsichtlich der Bauart, hinsichtlich der Größe und hinsichtlich der Ausrichtung mit dem in Figur 3 gezeigten Zuluftlüfterrad 14 identisches Fortlüftlüfterrad 19. Das Fortluft-Lüfterrad 19 ist zentriert innerhalb des Fortluft-Lüftergehäuses 7 angeordnet. Horizontal auf gleicher Höhe mit dem Fortluft-Lüfterrad 19 weist das Fortluft-Lüftergehäuse 7 auf der rechten Seite sowie das Gehäuse 3 auf seiner Rückseite jeweils eine Fortluftauslassöffnung 9 auf. An dieser Fortluftauslassöffnung 9 setzt ein Fortluftauslassrohr 20 an, das leicht nach rechts unten geneigt verläuft. Mittig auf der Oberseite des Fortluft-Lüftergehäuses 7 ist eine Fortluftzuführungsöffnung 18 vorgesehen, deren Breite ungefähr einem Drittel der Gesamtbreite des Fortluft-Lüftergehäuses 7 entspricht. Zwischen dem linken und dem rechten Ende der Unterseite des Plattenwärmetauschers 2 und der linken und rechten Seite der Oberseite des Fortluft-Lüftergehäuses 7 ist der Trichter 10 angeordnet.

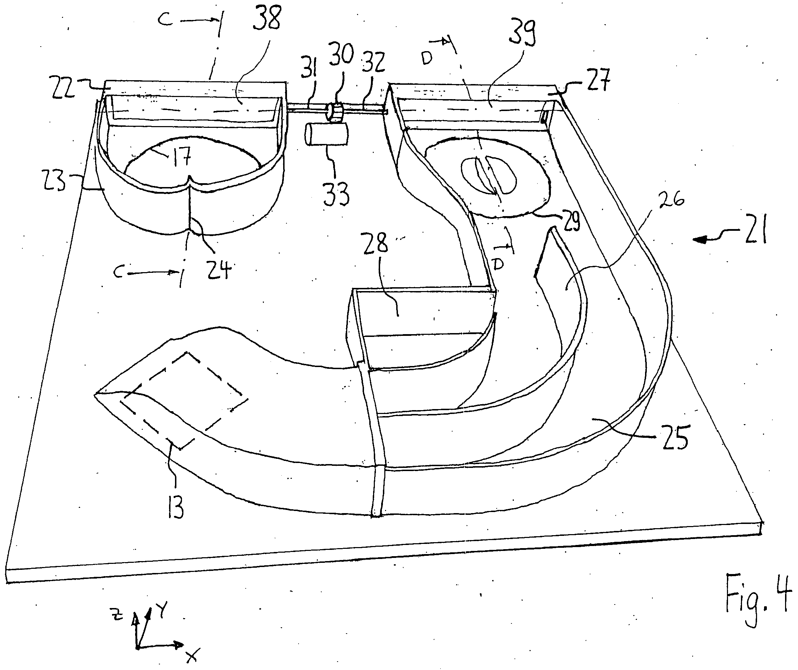

Figur 4 zeigt eine schematische, perspektivische Darstellung einer auf die Vorderseite des Gehäuses 3 der Luftaustauschvorrichtung 1 aufbringbaren Abdeckplatte 21 gemäß dem Ausführungsbeispiel.

Die Begriffe "unten" und "oben" werden hier so zur Beschreibung verwendet, wie es in Figur 4 mittels der z-Achse eines Koordinatensystems veranschaulicht ist. Die Begriffe "vorne" und "hinten" werden hier in Richtung der y-Achse gesehen verwendet. Die Begriffe "rechts" und "links" werden in Richtung der x-Achse gesehen verwendet.

Die Abdeckplatte 21 aus vorzugsweise nichtdurchlässigem Kunststoff ist dabei quadratisch geformt und so dimensioniert, daß sie beim Aufbringen auf die Vorderseite des Gehäuses 3 auf der unteren, auf der oberen, auf der linken sowie auf der rechten Seite mit der Umrandung des Gehäuses 3 vollständig abschließt. Im folgenden wird die Abdeckplatte 21 in auf dem Gehäuse 3 aufgesetzten Zustand beschrieben, wobei die Oberseite der Abdeckplatte 21 betrachtet wird.

Über der Abluft-Rundfilterpatrone 6 ist die kreisförmige Ablufteinlassöffnung 17 angeordnet, die hinsichtlich ihres Durchmessers mit dem Innendurchmesser der Abluft-Rundfilterpatrone 6 übereinstimmt. Auf der Oberseite der Abdeckplatte 21 ist die Ablufteinlassöffnung 17 von einem Ablufteinlassbereich 22 und von einer Abluftzuführung 23 umgeben.

Der Ablufteinlassbereich 22 beinhaltet eine rechteckige, schlitzartige Durchgangsöffnung und weist die gleiche Höhe wie die Abluftzuführung 23 sowie eine Breite auf, die den Durchmesser der Ablufteinlassöffnung 17 geringfügig übersteigt. Der Ablufteinlassbereich 22 schließt mit der oberen Kante der Abdeckplatte 21 ab. Die Durchgangsöffnung des Ablufteinlassbereichs 22 ist in Richtung der Ablufteinlassöffnung 17 gerichtet.

Die Abluftzuführung 23 ist als dünne Wand auf der Oberseite der Abdeckplatte 21 ausgebildet, die eine Höhe von ungefähr einem Drittel des Durchmessers der Ablufteinlassöffnung 17 aufweist. Die Abluftzuführung 23 setzt an der linken sowie an der rechten Seite des Ablufteinlassbereichs 22 an und verläuft bis ungefähr auf der Höhe der breitesten Stelle der Ablufteinlassöffnung 17 senkrecht nach vorne. Ab dort ist die Abluftzuführung 23 gebogen und verläuft in einem konstanten, geringen Abstand zu der kreisförmigen Ablufteinlassöffnung 17. Unterhalb der Mitte der Ablufteinlassöffnung 17 weist die Abluftzuführung 23 eine etwas nach hinten gerichtete Leitrippe 24 auf.

Im Betrieb der Luftaustauschvorrichtung 1 ist oben auf dem Ablufteinlassbereich 22 sowie oben auf der Abluftzuführung 23 eine Abdeckung aufgebracht, die mit dem Ablufteinlassbereich 22 sowie mit der Abluftzuführung 23 dicht abschließt.

Des weiteren ist auf der Abdeckplatte 21 ein Zuluftführungskanal 25 vorgesehen, der in einem linken vorderen Bereich der Oberseite der Abdeckplatte 21 oberhalb der Zuluftauslassöffnung 13 ansetzt, von dort ausgehend nach rechts verläuft, eine Biegung um 90° nach hinten aufweist und von dort ab parallel zum rechten Rand der Abdeckplatte 21 nach hinten verläuft. Der Zuluftführungskanal 25 wird in dem rechten hinteren Bereich der Oberseite der Abdeckplatte 21 durch einen Zuluftauslassbereich 27 begrenzt.

Von der Zuluftauslassöffnung 13 bis zu der Mitte der Vorderseite der Abdeckplatte 21 ist der Zuluftführungskanal 25 in Figur 4 auf der vorderen, auf der hinteren sowie auf der oberen Seite durch jeweils eine Abdeckung verschlossen. Ab der Mitte der Vorderseite bis zu dem Zuluftauslassbereich 27 ist der Zuluftführungskanal 25 ohne seine obere Abdeckung dargestellt. Diese obere Abdeckung wird vor dem Betrieb der Luftaustauschvorrichtung 1 derart auf den Zuluftführungskanal 25 aufgesetzt, daß sie mit seinen Seitenwänden dicht abschließt. Die Breite des Zuluftführungskanals 25 bleibt beinahe über seine gesamte Länge konstant und ist geringfügig größer ausgebildet als die Breite der Zuluftauslassöffnung 13. Die Höhe des Zuluftführungskanals 25 entspricht der Höhe der Wand der Abluftzuführung 23. Unmittelbar vor dem Zuluftauslassbereich 27 verbreitert sich der Zuluftführungskanal 25 derart, daß die linke sowie die rechte Wand des Zuluftführungskanals 25 jeweils mit dem linken bzw. mit dem rechten Rand des Zuluftauslassbereichs 27 abschließen.

Zwischen der Mitte der Vorderseite und dem mittleren, rechten Bereich des Zuluftführungskanals 25 verläuft eine Zwischenwandung 26 mittig zwischen der linken und der rechten Wand des Zuluftführungskanals 25. Diese gliedert den Zuluftführungskanal 25 in diesem Bereich in einen linken und in einen rechten Teilkanal. Die Zwischenwandung 26 verläuft dabei in etwa mit der gleichen Krümmung wie die linke bzw. die rechte Wand des Zuluftführungskanals 25.

Ein Duftstoffbehältnis 28 ist oberhalb der hinteren Wand des Zuluftführungskanals 25 im Bereich ihrer Biegung angeordnet. Das Duftstoffbehältnis 28 ist über eine schmale Öffnung mit dem linken Teilkanal des Zuluftführungskanals 25 verbunden. Im vorliegenden Ausführungsbeispiel bildet ein Teil der hinteren Wand des Zuluftführungskanals 25 gleichzeitig die untere rechte Wand des Duftstoffbehältnisses 28. Die linke Wand des Duftstoffbehältnisses 28 verläuft ausgehend von dem waagerechten Teil des Zuluftführungskanals 25 nach oben. Die obere Wand des Duftstoffbehältnisses 28 verläuft waagerecht und wird auf der linken Seite durch die linke Wand des Duftstoffbehältnisses 28 und auf der rechten Seite von der linken Wand des Zuluftführungskanals 25 begrenzt.

Der Zuluftauslassbereich 27 entspricht hinsichtlich der Größe und der Form dem Ablufteinlassbereich 22. Der Zuluftauslassbereich 27 schließt mit der Hinterseite der Abdeckplatte 21 auf ihrer rechten Seite ab und weist eine rechteckige, schlitzartige Öffnung auf, die im Ausführungsbeispiel nach hinten gerichtet ist.

In dem sich verbreiternden Bereich des Zuluftführungskanals 25 unmittelbar vor dem Zuluftauslassbereich 27 ist eine kreisförmige Wartungsluke 29 in der Abdeckplatte 21 vorgesehen. Diese befindet sich direkt oberhalb der Außenluft-Rundfilterpatrone 5, die mit einem in der Wartungsluke 29 vorgesehenen Griff für Wartungsarbeiten sowie für den Austausch der Außenluft-Rundfilterpatrone 5 aus der Abdeckplatte 21 herausgenommen und wieder eingesetzt werden kann.

Der Ablufteinlassbereich 22 weist eine Abluftklappe 38 auf, mit der die schlitzartige Öffnung des Ablufteinlassbereichs 22 geöffnet und verschlossen werden kann. Die Abluftklappe 38 ist dabei innerhalb der schlitzartigen Öffnung des Ablufteinlassbereichs 22 über eine Abluftklappen-Drehachse 31 drehbar. Diese Abluftklappen-Drehachse 31 verläuft inmitten der schlitzartigen Öffnung parallel zu der waagerechten Seite der Abdeckplatte 21. In Figur 4 ist eine Schnittlinie C-C vorgesehen, die von vorne nach hinten durch die Leitrippe 24 der Abluftzuführung 23, durch die Ablufteinlassöffnung 17 sowie durch den Ablufteinlassbereich 22 mit der Abluftklappe 38 verläuft.

Der Zuluftauslassbereich weist eine Zuluftklappe 39 auf, mit der die schlitzartige Öffnung des Zuluftauslassbereichs 27 geöffnet und verschlossen werden kann. Die Zuluftklappe 39 ist mittels einer Zuluftklappen-Drehachse 32 innerhalb der schlitzartigen Öffnung des Zuluftauslassbereichs 27 drehbar, wobei die Zuluftklappen-Drehachse 32 als Verlängerung der Abluftklappen-Drehachse 31 ausgebildet ist und parallel zu der waagerechten Seite der Abdeckplatte 21 inmitten durch die schlitzartige Öffnung des Zuluftauslassbereichs 27 verläuft. Auf der rechten, hinteren Seite der Abdeckplatte 21 in Figur 4 ist eine Schnittlinie D-D dargestellt, die ausgehend von knapp oberhalb dem Ende der Zwischenwandung 26 durch die Wartungsluke 29 und durch den Zuluftauslassbereich 27 mit der Zuluftklappe 39 verläuft.

Die Abluftklappen-Drehachse 31 verläuft rechts aus dem Ablufteinlassbereich 22 heraus, die Zuluftklappen-Drehachse 32 verläuft links aus dem Zuluftauslassbereich 27 heraus. In der Mitte zwischen dem Ablufteinlassbereich 22 und dem Zuluftauslassbereich 27 ist ein Ritzel 30 angeordnet, das mit der Abluftklappen-Drehachse 31 fest verbunden ist und in das die Zuluftklappen-Drehachse eingreift. Dieses Ritzel 30 ist über einen im Ausführungsbeispiel direkt davor angeordneten Elektromotor 33 antreibbar. Dieser Elektromotor 33 kann dazu ein in Figur 4 nicht dargestelltes Antriebsritzel aufweisen, das durch den Elektromotor 33 bewegt wird und das diese Bewegungen an das Ritzel 30 weitergibt.

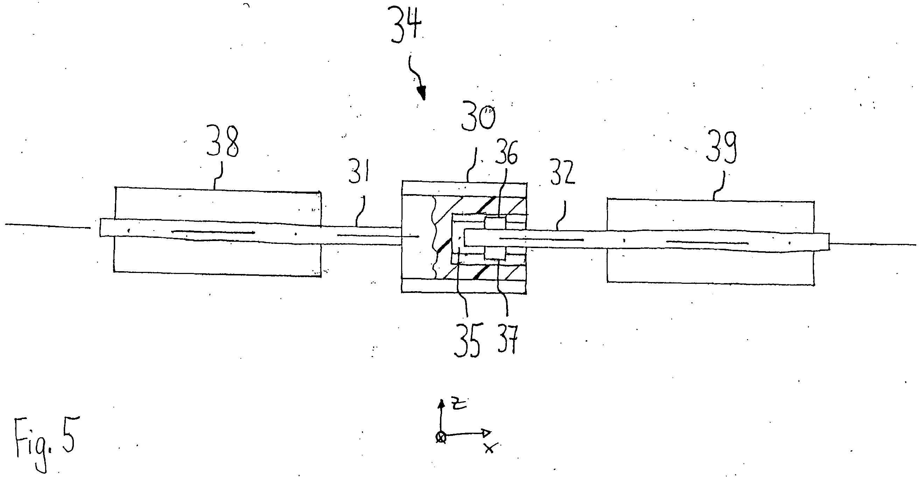

Figur 5 zeigt eine Prinzipskizze einer Klappenbetätigungsmechanik 34 der Abluftklappe 38 und der Zuluftklappe 39 gemäß dem Ausführungsbeispiel.

Die Abluftklappe 38 sowie die Abluftklappen-Drehachse 31 sind in Figur 5 in einer Draufsicht dargestellt. Gemäß der Darstellung in Figur 5 ist die rechteckig geformte Abluftklappe 38 mittels der waagerecht verlaufenden Abluftklappen-Drehachse 31 fest mit dem Ritzel 30 verbunden.

Das Ritzel 30 ist in Figur 5 in Form einer teilweise aufgeschnittenen Draufsicht gezeigt. Bei dieser Darstellung wird deutlich, daß das Kunststoff aufweisende Ritzel 30 auf seiner Außenseite jeweils Zähne aufweist. Des weiteren ist in Figur 5 ersichtlich, daß das Ritzel 30 eine mittig auf seiner rechten Seite angeordnete innere Aussparung 35 beinhaltet. Diese innere Aussparung 35 verläuft von der rechten Seite des Ritzels 30 bis knapp über seine Mitte und weist wenigstens einen radial angeordneten Anschlagbereich auf.

Die Zuluftklappe 39, die Zuluftklappen-Drehachse 32, ein erster Vorsprung 36 und ein zweiter Vorsprung 37 sind in Figur 5 in einer Draufsicht dargestellt. Der erste Vorsprung 36 sowie der zweite Vorsprung 37 der Zuluftklappen-Drehachse 32 stehen mit der inneren Aussparung 35 des Ritzels 30 in Eingriff. Dieser erste Vorsprung 36 sowie dieser zweite Vorsprung 37 sind an der linken Seite der in die innere Aussparung 35 hineinverlaufenden Zuluftklappen-Drehachse 32 ausgebildet, wobei der erste Vorsprung 36 in Figur 5 nach oben zeigt und wobei der zweite Vorsprung 37 nach unten zeigt. Die Zuluftklappen-Drehachse 32 ist in der Verlängerung der Abluftklappen-Drehachse 31 angeordnet und fest mit der rechteckig geformten Zuluftklappe 39 verbunden. Die Abluftklappe 38, das Ritzel 30 sowie die Zuluftklappe 39 sind in Figur 5 um die Abluftklappen-Drehachse 31 bzw. um die Zuluftklappen-Drehachse 32 drehbar ausgebildet.

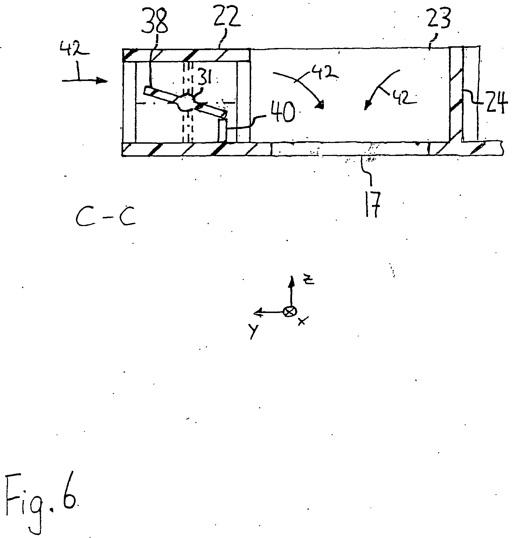

Figur 6 zeigt eine schematische Darstellung eines Schnitts der Abdeckplatte 21 entlang der in Figur 4 gezeigten Schnittlinie C-C gemäß dem Ausführungsbeispiel.

In dieser Schnittdarstellung ist die Grundplatte der Abdeckplatte 21 waagerecht dargestellt, wobei die Ablufteinlassöffnung 17 in einem mittleren Bereich erkennbar ist. In einem linken Bereich der Schnittdarstellung ist der Ablufteinlassbereich 22 mit der Abluftklappe 38 erkennbar. Der Ablufteinlassbereich 22 ist dabei als waagerechte Öffnung dargestellt, in deren Mitte die Abluftklappe 38 angeordnet ist. Diese Abluftklappe 38 ist um die Abluftklappen-Drehachse 31 drehbar, die senkrecht zur Schnittrichtung verläuft. Die Abluftklappe 38 ist um einen Winkel von ungefähr 110° gegenüber ihrer Ausgangsstellung gedreht und liegt mit ihrem rechten Ende auf einem auf der Grundplatte der Abdeckplatte 21 ausgebildeten Abluftklappenanschlag 40 auf. In der Grundstellung verläuft die Abluftklappe 38 senkrecht zur Öffnung des Ablufteinlassbereichs 22 und verschließt diese Öffnung vollständig.

Die Abluftzuführung 23 ist auf der rechten Seite an den Ablufteinlassbereich 22 anschließend dargestellt und beinhaltet die auf der rechten Seite der Schnittdarstellung gezeigte, geschnittene Leitrippe 24. Der Ablufteinlassbereich 22 sowie die Abluftzuführung 23 weisen in dem Ausführungsbeispiel die gleiche Höhe auf. Auf die Abluftzuführung 23 ist von oben eine Abdeckung aufbringbar, die auf der linken Seite mit dem rechten Rand des Ablufteinlassbereichs 22 abschließt und über die gesamte Oberseite der Abluftzuführung 23 ausgebildet ist.

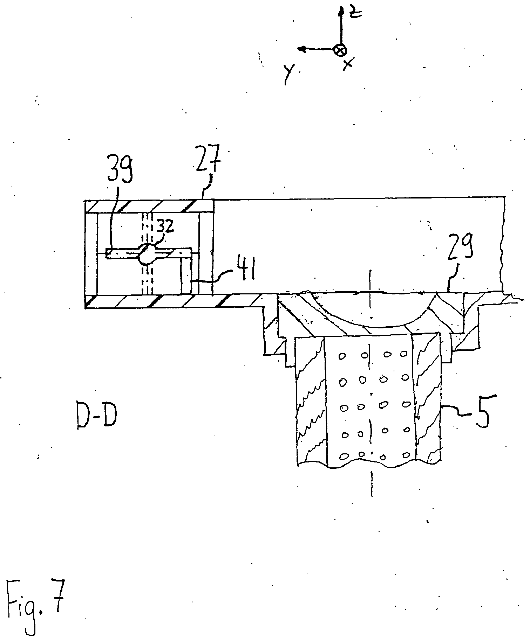

Figur 7 zeigt eine schematische Darstellung eines Schnitts der Abdeckplatte 21 entlang der in Figur 4 gezeigten Schnittlinie D-D gemäß dem Ausführungsbeispiel.

In einem linken Bereich der Schnittdarstellung in Figur 7 ist der Zuluftauslassbereich 27 dargestellt. Dieser entspricht hinsichtlich der Form und der Größe dem in Figur 6 dargestellten Ablufteinlassbereich 22. Innerhalb des Zuluftauslassbereichs 27 ist die Zuluftklappe 39 angeordnet, die drehbar um eine senkrecht zur Schnittrichtung verlaufende Zuluftklappen-Drehachse 32 ausgebildet ist. In Figur 7 ist die Zuluftklappe 39 waagrecht ausgerichtet, wobei ihr rechtes Ende auf einem im rechten, unteren Bereich des Zuluftauslassbereichs 27 angeordneten Zuluftklappenanschlag aufliegt. Die Zuluftklappe 39 ist ausgehend von ihrer Schließstellung um einen Winkel von 90° nach rechts gedreht. Die Zuluftklappe 39 schließt in ihrer Schließstellung die schlitzartige Öffnung des Zuluftauslassbereichs 27 senkrecht ab.

In einem rechten Bereich der Schnittdarstellung in Figur 7 ist die in die Grundplatte der Abdeckplatte 21 eingesetzte Wartungsluke 29 dargestellt. Die Wartungsluke 29 weist dabei auf ihrer Oberseite mittig einen bogenförmigen aus ihr ausgeschnittenen Griff auf. Unterhalb der Wartungsluke 29 befindet sich die Außenluft-Rundfilterpatrone 5, deren Mantelbereich auf der linken und auf der rechten Seite und deren Innenbereich mittig nach unten verlaufend dargestellt sind. Die Wartungsluke 29 ist dabei so dimensioniert, daß der obere Bereich der Außenluft-Rundfilterpatrone 5 an der Unterseite in die Wartungsluke 29 eingepaßt ist.

Auf der rechten Seite an den Zuluftauslassbereich 27 angrenzend ist die in Figur 4 dargestellte, rechte Wand des Zuluftführungskanals 25 dargestellt. Diese sowie der Zuluftauslassbereich 27 weisen in der Schnittdarstellung in Figur 7 die gleiche Höhe auf. Auf den Zuluftführungskanal 25 kann von oben eine mit der rechten Wand sowie mit der linken Wand abschließende Deckplatte aufgebracht werden, die sich bis zu dem Zuluftauslassbereich 27 erstreckt und den Zuluftführungskanal 25 auf allen Seiten dicht abschließt.

Wie in Figur 1 und in Figur 2 dargestellt, wird durch den Zuluftansaugradiallüfter 12 bzw. durch dessen Zuluftlüfterrad 14 kalte Außenluft angesaugt, die durch das in Figur 2 gezeigte Außenluftzufuhrrohr 16 auf der Rückseite des Gehäuses 3 in die Luftaustauschvorrichtung 1 eintritt. Die Außenluft strömt dabei zunächst in den Innenraum der Außenluftrundfilterpatrone 5. Von dort aus strömt sie radial durch den Filtermaterial aufweisenden Mantelbereich der Außenluftrundfilterpatrone 5. Dabei kann die Außenluft auf der gesamten Mantelfläche der Außenluftrundfilterpatrone 5 auf einer Vielzahl von unterschiedlichen Luftwegen hindurchströmen. Anschließend strömt die derart gereinigte Außenluft durch die in Figur 2 besonders gut erkennbaren Luftführungen des Plattenwärmetauschers 2 und wird dabei erwärmt. Anschließend fließt die erwärmte Luft durch die Zuluftauslassöffnung 13 in den in Figur 4 besonders gut erkennbaren Zuluftführungskanal 25 der Abdeckplatte 21. Diese Zuluft teilt sich beim Durchlaufen des Zuluftführungskanals 25 in einen links der Zwischenwandung 26 sowie in einen rechts der Zwischenwandung 26 verlaufenden Teil. Der links der Zwischenwandung 26 verlaufende Luftstrom strömt an dem Duftstoffbehältnis 28 vorbei und nimmt eine definierte, geringe Menge dieses Duftstoffs auf. Oberhalb des Endes der Zwischenwandung 26 vereinigen sich die beiden Zuluftströme zu einem gemeinsamen Zuluftstrom. Dieser Zuluftstrom verläßt die Abdeckplatte 21 durch den in Figur 7 dargestellten Zuluftauslassbereich 27, wobei die Zuluftklappe 39 insbesondere waagrecht gestellt ist. Der so durch den Plattenwärmetauscher 2 erwärmte Zuluftstrom strömt dann in den Innenraum des Gebäudes.

Das Vorsehen des sich über zwei Seiten des Plattenwärmetauschers 2 erstreckenden Zuluftführungskanal 25, der von der Zuluft durchströmt wird, bewirkt eine Schalldämpfung der durch den Zuluftansaugradiallüfter 12 verursachten Drehgeräusche, die an dem Zuluftauslassbereich 27 deutlich reduziert sind.

Wie in Figur 1 sowie in Figur 3 ersichtlich ist, saugt der Fortluft-Radiallüfter 8 bzw. dessen Fortluft-Lüfterrad 19 gleichzeitige zu dem vorstehend beschriebenen Vorgang warme, verbrauchte Abluft aus dem Gebäudeinneren an. Diese warme Abluft strömt durch den in Figur 4 sowie in Figur 6 besonders gut erkennbaren Ablufteinlassbereich 22 zunächst in die Abluftzuführung 23. Dabei ist die Abluftklappe 38, wie in Figur 6 dargestellt, in Bezug auf die Strömungsrichtung der Abluft leicht nach unten geneigt. Dadurch wird eine vorteilhafte Strömungsrichtung der Abluft in Richtung der Ablufteinlassöffnung 17 erreicht. Durch die in der Abluftzuführung vorgesehenen Leitrippe 24 wird darüberhinaus ein günstiges Strömungsverhalten der Abluft in die Ablufteinlassöffnung 17 und in die unter der Ablufteinlassöffnung 17 gelegene Abluft-Rundfilterpatrone 6 gewährleistet. Die Abluft tritt nun durch die Ablufteinlassöffnung 17 in das Gehäuse 3 ein und zwar in das Innere der Abluft-Rundfilterpatrone 6. Dann durchströmt die Abluft den Filtermaterial aufweisenden Mantelbereich der Abluft-Rundfilterpatrone 6, insbesondere radial, in verschiedene Richtungen und wird dabei gereinigt. Anschließend strömt die Abluft, wie in Figur 3 ersichtlich, durch die Luftführungen des Plattenwärmetauschers 2 und gibt dabei Wärme ab, wird also gekühlt. Danach gelangt die Abluft durch den Trichter 10 sowie durch die Förtluft-Zuführungsöffnung 18 in das Fortluft-Lüftergehäuse 7. Von dort aus wird die nun als Fortluft bezeichnete Abluft in Folge der Bewegung der Schaufeln des Fortluft-Lüfterrads 19 sowie in Folge der Druckdifferenz, die zwischen dem Innenraum des Fortluft-Lüfterrads 19 und dem außerhalb der Fortluftauslassöffnung 9 gelegenen Bereich herrscht durch die Fortluftauslassöffnung 9 der Rückseite des Gehäuses 3 aus der Luftaustauschvorrichtung 1 sowie durch das Fortluft-Auslassrohr 20 auf eine Außenseite des Gebäudes befördert.

Gemäß Figur 5 ist eine Klappenbetätigungsmechanik 34 mit einem einzigen Elektromotor für die Steuerung der Abluftklappe 38 sowie für die Steuerung der Zuluftklappe 39 vorgesehen. Dabei wird das Ritzel 30 durch ein Antriebsritzel des Elektromotors 33 betätigt.

Durch diese Klappenbetätigungsmechanik 34 wird die jeweils gewünschte Stellung der Abluftklappe 38 und der Zuluftklappe 39 eingestellt. Im erfindungsgemäßen Betrieb der Luftaustauschvorrichtung 1 sind dies die vorstehend mit Bezug auf Figur 6 und Figur 7 beschriebenen, jeweils voneinander verschiedenen Öffnungsstellungen der Abluftklappe 38 und der Zuluftklappe 39. In dem Ritzel 30 ist ein Spiel zwischen der Abluftklappen-Drehachse 31 und der Zuluftklappen-Drehachse 32 vorgesehen, durch das mit genau einer Drehung des Ritzels 30 ausgehend von der jeweils selben Schließstellung der Abluftklappe 38 und der Zuluftklappe 39 die verschiedenen Öffnungsstellungen erreicht werden können. Soll keine Abluft in die Luftaustauschvorrichtung 1 gelangen können bzw. soll keine Zuluft aus der Luftaustauschvorrichtung 1 in das Gebäudeinnere gelangen können, so werden die Abluftklappe 38 und die Zuluftklappe 39 durch genau eine Drehung des Ritzels 30, ausgehend von ihren Öffnungsstellungen in ihre jeweilige Schließstellung gedreht. Dann steht die Luftaustauschvorrichtung 1 still und ist vor äußeren Einflüssen geschützt.

Zusätzlich dazu kann ein Energiespeicher, wie eine Batterie, ein Kondensator, eine Spule oder eine Feder vorgesehen sein, der im Falle eines Stromausfalls oder einer Unterbrechung der Energiezufuhr zu der Luftaustauschvorrichtung 1 mit Hilfe einer zusätzlich vorgesehenen Ansteuerschaltung den Elektromotor so betätigt, daß unter Nutzung der im Energiespeicher gespeicherten Energie die Abluftklappe 38 und die Zuluftklappe 39 in ihre jeweilige Schließstellung bewegt werden.

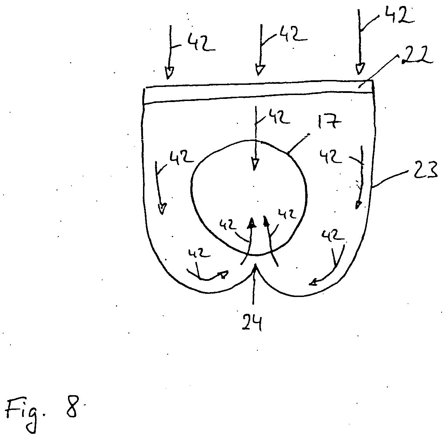

In Figur 8 sind die Strömungsrichtungen der Abluft anhand der Strömungspfeile 42 veranschaulicht. Dabei tritt die Abluft aus dem Gebäudeinneren durch den Ablufteinlassbereich 22 in die Abluftzuführung 23 ein. Die Strömungsrichtung ist dabei senkrecht zur schlitzartigen Öffnung des Ablufteinlassbereichs 22. Die mittig in den Ablufteinlassbereich 22 einströmende Abluft wird direkt in die Ablufteinlassöffnung 17 gelenkt. Durch die im unteren Bereich von Figur 8 dargestellte, bogenartige Krümmung der Abluftführung 23 sowie durch die Leitrippe 24 wird die am Rand der Abluftführung 23 strömende Abluft vorteilhafterweise in die Ablufteinlassöffnung 17 hineingelenkt.

## Patentansprüche

1. Luftaustauschvorrichtung, insbesondere für einen Innenraum eines Gebäudes, mit einem Gehäuse (3) und mit einem im Gehäuse (3) angeordneten elektrischen Lüfter (12) zur Förderung von Zuluft in den Innenraum und mit einer Abdeckplatte (21), die mit einer Umrandung des Gehäuses (3) abschließt, wobei auf der Abdeckplatte (21) ein Zuluftführungskanal (25) vorgesehen ist, der sich entlang des Bereichs wenigstens einer Gehäuseseite erstreckt, wobei der Zuluftführungskanal (25) eine Zwischenwandung (26) aufweist, die im Wesentlichen parallel zur Luftströmungsrichtung in dem Zuluftführungskanal (25) verläuft, wobei der Zuluftführungskanal (25) bogenförmig ausgebildet ist und sich entlang der Bereiche von zwei aneinander grenzenden Gehäuseseiten erstreckt.

2. Luftaustauschvorrichtung nach Anspruch 1, wobei der Zuluftführungskanal eine Biegung um 90 Grad aufweist.

3. Luftaustauschvorrichtung nach Anspruch 2, wobei der Zuluftführungskanal 25 in einem vorderen Bereich der Abdeckplatte (21) ansetzt und wobei der Zuluftführungskanal von der Biegung um 90 Grad ab parallel zu einem Rand der Abdeckplatte (21) verläuft.

4. Luftaustauschvorrichtung nach Anspruch 1 oder 2,
**dadurch gekennzeichnet, dass**
in dem Zuluftführungskanal (25) wenigstens eine UV-Lampe zum Abtöten von in der Zuluft vorhandenen Keimen angeordnet ist.

5. Luftaustauschvorrichtung nach Anspruch 1 mit einem insbesondere bei einer Abluft-Filtervorrichtung (6) gelegenen Ablufteinlassbereich (22), wobei an dem Ablufteinlassbereich (22) eine Abluftzuführung (23) mit einer Leitrippe (24) zur Führung der Abluft in den Innenbereich der Luftaustauschvorrichtung (1) vorgesehen ist.

6. Luftaustauschvorrichtung nach Anspruch 1, insbesondere für einen Innenraum eines Gebäudes, mit einem Gehäuse (3) und mit einem im Gehäuse (3) angeordneten elektrischen Lüfter (12) zur Förderung von Zuluft in den Innenraum, wobei am Gehäuse (3) ein Zuluftführungskanal (25) vorgesehen ist, der sich entlang des Bereichs wenigstens einer Gehäuseseite erstreckt, wobei in dem Zuluftführungskanal (25) wenigstens eine UV-Lampe zum Abtöten von in der Zuluft vorhandenen Keimen angeordnet ist.

7. Luftaustauschvorrichtung nach Anspruch 6,
**dadurch gekennzeichnet, dass**
in einem Bereich des Zuluftführungskanals (25) ein mit einem Duftstoff gefülltes Behältnis (28) vorgesehen ist, aus dem der Duftstoff an die im Zuluftführungskanal (25) vorbeiströmende Luft abgebbar ist.

8. Luftaustauschvorrichtung nach Anspruch 6,
**dadurch gekennzeichnet, dass**
in einem Bereich des Zuluftführungskanals (25) ein Zerstäuber zum Zerstäuben von separat zugeleitetem Wasser und/oder ein Keramikzylinder zum Verdampfen von Duftstoffen, insbesondere von Duftöl, angeordnet ist.

## Claims

1. Air exchange device, in particular for an inner room of a building, with a housing (3) and with an electric ventilator (12) that is arranged in the housing (3) for the conveyance of incoming air into the inner room, and with a cover plate (21) which terminates with a boundary of the casing (3), wherein a guiding channel for incoming air (25) is provided on the cover plate (21), which extends along the area of at least one housing side, wherein the guiding channel for incoming air (25) comprises an intermediate wall (26) that runs essentially in parallel to the direction of air current in the guiding channel for incoming air (25), wherein the guiding channel for incoming air (25) is shaped in the form of an arc and extends along the areas of two mutually adjacent housing sides.

2. Air exchange device according to claim 1, wherein the guiding channel for incoming air comprises a turn of 90 degrees.

3. Air exchange device according to claim 2, wherein the guiding channel for incoming air (25) begins in a front portion of the cover plate (21) and wherein the guiding channel for incoming air runs in parallel to a border of the over plate (21) from the turn of 90 degrees onwards.

4. Air exchange device according to claim 1 or claim 2, **characterized in that**
at least one UV lamp is provided in the guiding channel for incoming air (25) to kill germs that are present in the incoming air.

5. Air exchange device according to claim 1, with an exhaust air inlet area (22) that is in particular located at an exhaust air filtration device (6), wherein an exhaust air supply (23) with a guiding fin (24) is provided at the exhaust air inlet area (22) for guiding the exhaust air.

6. Air exchange device according to claim 1, in particular for an inner room of a building, with a housing (3) and an electric ventilator (12) for the conveyance of incoming air into the inner room, the electric ventilator (12) being arranged in the housing (3), wherein a guiding channel for incoming air (25) is provided at the housing (3), which extends along the area of at least one housing side, wherein at least one UV lamp is provided in the guiding channel for incoming air (25) for killing germs which are present in the incoming air.

7. Air exchange device according to claim 6,
**characterized in that**
in an area of the guiding channel for incoming air (25) a fragrance filled container (28) is provided, from which the fragrance is releasable into the stream of passing air that circulates in the guiding channel for incoming air (25).

8. Air exchange device according to claim 6,
**characterized in that**
a nebulizer for nebulizing separately adduced water and/or a ceramic cylinder for the evaporation of fragrances, in particular of fragrance oil, is arranged in an area of the guiding channel for incoming air (25).

## Revendications

1. Dispositif de renouvellement d'air, en particulier pour une chambre intérieure d'un bâtiment, avec une boîte (3) et un ventilateur électrique (12) qui est arrangé dans la boîte (3) pour le transport d'air entrant dans la chambre intérieure, et avec une plaque de couverture (21) qui termine avec une bordure de la boîte (3), dans lequel un conduit d'air entrant (25) est pourvu sur la plaque de couverture (21) qui s'étend le long de la zone d'au moins un côté de boîte, dans lequel le conduit d'air entrant (25) comprend une paroi intermédiaire (26), qui s'étend sensiblement en parallèle à la direction de courant d'air dans le conduit d'air entrant (25), dans lequel le conduit d'air entrant (25) est dessiné en forme d'arc et s'étend le long des zones de deux côtés de boîte avoisinants.

2. Dispositif de renouvellement d'air selon revendication 1, dans lequel le conduit d'air entrant comprend une inflexion à 90 degrés.

3. Dispositif de renouvellement d'air selon revendication 2, dans lequel le conduit d'air entrant (25) commence dans une zone antérieure de la plaque de couverture (21), et le conduit d'air entrant (25) s'étend en parallèle avec une bordure de la plaque de couverture (21) après l'inflexion à 90 degrés.

4. Dispositif de renouvellement d'air selon revendication 1 ou 2, **caractérisé en ce que** dans le conduit d'air entrant (25) au moins une lampe U.V. est pourvue pour détruire des germes présents dans l'air entrant.

5. Dispositif de renouvellement d'air selon revendication 1, avec une zone d'entrée d'air sortant (22) qui est situé en particulier près d'un dispositif de filtration d'air sortant (6), dans lequel une alimentation d'air sortant (23) avec une nervure de guidage (24) pour guider le courant d'air sortant dedans la zone intérieure du dispositif de renouvellement d'air (1) est pourvu à la zone d'entrée d'air sortant (22).

6. Dispositif de renouvellement d'air selon revendication 1, en particulier pour une chambre intérieure d'un bâtiment, avec une boîte (3) et un ventilateur électrique (12) qui est arrangé dans la boîte (3) pour transporter de l'air entrant dans la chambre intérieure, dans lequel un conduit de guidage d'air entrant (25) est pourvu à la boîte, qui s'étend le long la zone d'au moins un côté de boîte, dans lequel au moins une lampe U.V. est pourvue dans le conduit de guidage d'air entrant (25) pour détruire des germes présents dans l'air entrant.

7. Dispositif de renouvellement d'air selon revendication 6, **caractérisé en ce qu'**il y a, dans une zone du conduit de guidage d'air entrant (25), un récipient (28) remplit avec un parfum, qui peut être transmit à l'air passant qui circule dans le conduit de guidage d'air entrant (25).

8. Dispositif de renouvellement d'air selon revendication 6, **caractérisé en ce que**, dans une zone de conduit de guidage d'air entrant (25), il y a un nébuliseur pour nébuliser de l'eau séparément alimenté et/ou un cylindre céramique pour vaporiser de parfums, en particulier de l'huile parfumée.
